# EUROPÄISCHE PATENTSCHRIFT

(11) **EP 3 035 977 B1**
(45) Veröffentlichungstag und Bekanntmachung des Hinweises auf die Patenterteilung: **31.07.2019**
(21) Anmeldenummer: 14750723.0
(22) Anmeldetag: 12.08.2014
(51) Int. Cl.: A61M 5/142, A61M 5/145, A61M 37/00

(54) **TRANSDERMALES THERAPEUTISCHES SYSTEM MIT DRUCKERZEUGUNGSVORRICHTUNG**
TRANSDERMAL THERAPEUTIC SYSTEM WITH PRESSURE GENERATING DEVICE
SYSTÈME THÉRAPEUTIQUE TRANSDERMIQUE DOTÉ D'UN DISPOSITIF DE GÉNÉRATION DE PRESSION

(30) Priorität: 23.08.2013 EP 13181516
(43) Veröffentlichungstag der Anmeldung: 29.06.2016
(73) Patentinhaber: LTS Lohmann Therapie-Systeme AG, 56626 Andernach (DE)
(72) Erfinder: MOHR, Patrick, 53498 Bad Breisig (DE)
(74) Vertreter: Thämer, Wolfgang
(86) Internationale Anmeldenummer: PCT/EP2014/067238
(87) Internationale Veröffentlichungsnummer: WO 2015/024816

(56) Entgegenhaltungen:
- WO-A2-2011/071287
- WO-A2-2011/084951
- US-A- 5 848 991
- US-A1- 2011 172 601
- US-B1- 6 611 707

## Beschreibung

Die Erfindung betrifft ein transdermales therapeutisches System mit einem Systemträger, mit mindestens einem in den Systemträger eingebetteten Vorschubelement und mit mindestens einem zumindest bereichsweise elastisch verformbaren, in das Vorschubelement eingebetteten Wirkstoffreservoir, wobei auf der Anwendungsseite des transdermalen therapeutischen Systems der Systemträger das Vorschubelement und dieses das Wirkstoffreservoir umgibt und wobei das Vorschubelement einen mittels Flüssigkeitsaufnahme expandierbaren Quellkörper umfasst.

Aus der nachveröffentlichten EP 12 158 740.6 ist ein derartiges System bekannt. Ein transdermales therapeutisches System gemäß dem Oberbegriff des unabhängigen Anspruchs 1 ist aus der US 5,848,991 bekannt.

Der vorliegenden Erfindung liegt die Problemstellung zugrunde, die Wirkung des Vorschubelements zu verstärken.

Diese Problemstellung wird mit den Merkmalen des Hauptanspruchs gelöst. Dazu ist der Systemträger zumindest bereichsweise verformungssteif ausgebildet.

Weitere Einzelheiten der Erfindung ergeben sich aus den Unteransprüchen und den nachfolgenden Beschreibungen schematisch dargestellter Ausführungsbeispiele.
- Figur 1:: Transdermales therapeutisches System;
- Figur 2:: System nach dem Aufsetzen auf die Haut;
- Figur 3:: System bei der Abgabe des Wirkstoffes in die Haut;
- Figur 4:: System mit Hohlnadeln am Vorschubelement;
- Figur 5:: System mit biege- und torsionssteifer Druckplatte;
- Figur 6:: System mit das Wirkstoffreservoir durchdringenden hohlen Nadeln;
- Figur 7:: System wie Figur 6 mit hohlen Nadeln und semipermeabler Membran;
- Figur 8:: System mit integriertem Wasserreservoir;
- Figur 9:: System nach Figur 8 nach dem Öffnen des Wasserreservoirs.

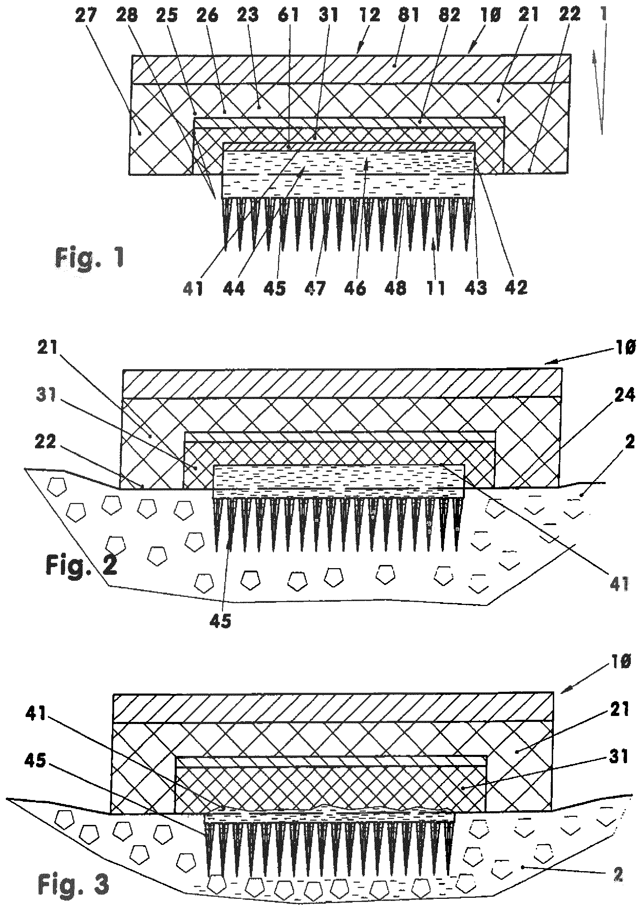

Die Figuren 1 - 3 zeigen ein transdermales therapeutisches System (10). Derartige Systeme (10) werden eingesetzt, um flüssige oder verflüssigbare Wirkstoffe in die Haut (2) eines Patienten einzubringen. Es hat eine der Haut (2) des Patienten zuwendbare Anwendungsseite (11) und eine dieser abgewandte Handhabungsseite (12).

Das transdermale therapeutische System (10) umfasst einen Systemträger (21), der beispielsweise mittels einer adhäsiven Schicht (22) auf der Haut (2) des Patienten befestigbar ist. Diese adhäsive Schicht (22) kann frei von Wirkstoffen sein. Sie kann an der der Haut (2) zugewandten Auflagefläche (24) auf einen Tragteil (23) des Systemträgers (21) aufgebracht sein. Es ist aber auch denkbar, den gesamten Systemträger (21) aus dem Werkstoff der adhäsiven Schicht (22) aufzubauen.

Die der Haut (2) abgewandte Seite des Systemträgers (21) trägt im Ausführungsbeispiel einen Abdeckungslayer (81). Bei einer Ausführung des Systemträgers (21) aus dem Werkstoff der adhäsiven Schicht (22) ist der Abdeckungslayer (81) mit dem Systemträger (21) verklebt.

Der Systemträger (21) ist als konkaver Körper aufgebaut. Er umfasst ein Deckenteil (26) sowie daran angrenzende Wandteile (27). Im Ausführungsbeispiel sind die Wandteile (27) doppelt so dick wie das Deckenteil (26). Im Ausführungsbeispiel beträgt die Dicke des Deckenteils (26) mehr als ein Drittel der Höhe des transdermalen therapeutischen Systems (10) oberhalb der Auflagefläche (24). Der Systemträger (21) hat damit ein hohes Widerstandsmoment gegen Ausbeulung des Deckenteils (26). Im Ausführungsbeispiel ist an der Innenseite des Deckenteils (26) ein zusätzlicher Schutzlayer (82) angeordnet. Die Höhe der Wandteile (27) in einer Richtung normal zur Auflagefläche (24) ist größer als zwei Drittel der Gesamthöhe des transdermalen therapeutischen Systems (10) oberhalb der Auflagefläche (24). Damit hat der Systemträger (21) ein hohes Widerstandsmoment sowohl gegen Biegung als auch gegen Torsion. Der Systemträger (21) ist somit verformungssteif ausgebildet, unter Berücksichtigung der für den Tragekomfort notwendigen Flexibilität.

Die Wandteile (27) und das Deckenteil (26) bilden eine Schale (25) und begrenzen fünfseitig einen Innenraum (28) des Systemträgers (21). Die Anbindung der Wandteile (27) an das Deckenteil (26) kann rechtwinklig sein. Im Bereich dieser Anbindung können Hohlkehlen ausgebildet sein. Der Innenraum (28) kann jedoch auch trichterförmig in Richtung der Anwendungsseite (11) geöffnet sein. Der von einem Wandteil (27) und einer vertikalen Ebene eingeschlossene Winkel ist z.B. kleiner oder gleich 45 Grad.

Im Innenraum (28) ist ein Vorschubelement (31) angeordnet. Dieses ist im Einbauzustand bündig mit der Auflagefläche (24) oder es steht über die Auflagefläche (24) in Richtung der Haut (2) über. Das Vorschubelement (31) besteht im Ausführungsbeispiel aus einem elastisch und/oder plastisch verformbaren Werkstoff. Die Grundwerkstoffe des Vorschubelements (31) sind beispielsweise vernetzte Polymere, die das Natriumsalz der Acrylsäure enthalten. Auch der Einsatz von Natriumpolyacrylat oder vernetzten Polyacrylamiden ist denkbar. Diese Werkstoffe sind hydrophil. Sie können - je nach Zusammensetzung und Art der Flüssigkeit - das 30-fache bis 800-fache ihrer Masse an Flüssigkeit aufnehmen. Beispielsweise wird die Aufnahmefähigkeit durch die elektrische Potentialdifferenz des Werkstoffs und der aufzunehmenden Flüssigkeit bestimmt. Im Ausführungsbeispiel wird als Flüssigkeit Wasser eingesetzt. Dieses kann destilliert oder demineralisiert sein.

Bei der Aufnahme von Flüssigkeit vergrößert sich das Volumen des Vorschubelements (31). Beispielsweise ist die Volumenzunahme proportional zum Volumen der aufgenommenen Flüssigkeit. Das Vorschubelement (31) ist daher im Folgenden auch als Quellkörper (31) bezeichnet.

Im Vorschubelement (31) ist ein Wirkstoffreservoir (41) eingebettet. Es steht über die Auflagefläche (24) aus diesem hervor. Das Wirkstoffreservoir (41) ist beutelartig ausgebildet und enthält eine oder mehrere Wirkstoffzubereitungen. Es ist aus einem elastisch verformbaren Werkstoff hergestellt.

Anstatt eines einzelnen Wirkstoffreservoirs (41) können auch mehrere Wirkstoffreservoire (41) im Vorschubelement (31) eingebettet sein. Sie können hierbei nebeneinander oder übereinander angeordnet sein. Beispielsweise sind die Wandungen (42) der Wirkstoffreservoire (41) semipermeabel oder derart ausgestattet, dass beispielsweise einzelne Wirkstoffe unmittelbar vor der Anwendung miteinander vermischt werden.

Zwischen dem Vorschubelement (31) und dem Wirkstoffreservoir (41) kann eine Zwischenschicht (61) angeordnet sein. Diese kann beispielsweise als Druckplatte (61) und/oder als wasseraufnehmende Schicht (61) ausgebildet sein oder die wirkstoffhaltige Schicht (41) wird durch eine beispielsweise Folie (z.B. PET) von dem Vorschubelement (31) getrennt.

Am Wirkstoffreservoir (41) ist weiterhin ein Nadelträger (43) mit einer Vielzahl von Nadeln (45) befestigt. Der Nadelträger (43) und die Nadeln (45) ragen auf der dem Systemträger (21) abgewandten Seite aus der Auflagefläche (24) heraus. Der Nadelträger (43) umfasst beispielsweise einen Verteilkanal (48) und verbindet den mit Wirkstoff (44) befüllten Innenraum (46) des Wirkstoffreservoirs (41) mit den Nadeln (45). Das Wirkstoffreservoir (41) kann auch ohne Verteilkanal (48) ausgeführt sein. Der Nadelträger (43) bildet dann eine Begrenzung des Innenraums (46) des Wirkstoffreservoirs (41).

Die einzelne Nadel (45) hat eine Länge zwischen 10 Mikrometern und einem Millimeter. Ihr Außendurchmesser beträgt zwischen zehn Mikrometer und 500 Mikrometern. Im Ausführungsbeispiel sind alle Nadeln (45) als Hohlnadeln ausgebildet. Der Durchmesser der Durchgangsbohrung (47) beträgt zwischen 3 Mikrometern und 80 Mikrometern. Die auf der Anwendungsseite (11) am Nadelträger (43) befestigten Nadeln (45) können auch als poröse Nadeln (45) oder als auflösbare Nadeln (45) ausgebildet sein. Die in den Figuren kegelförmig dargestellten Nadeln (45) können auch einen zylinderförmigen Schaft und eine kegelförmige Spitze aufweisen. Die Nadeln (45) verbinden damit den Innenraum (46) des Wirkstoffreservoirs (41) mit der Umgebung (1).

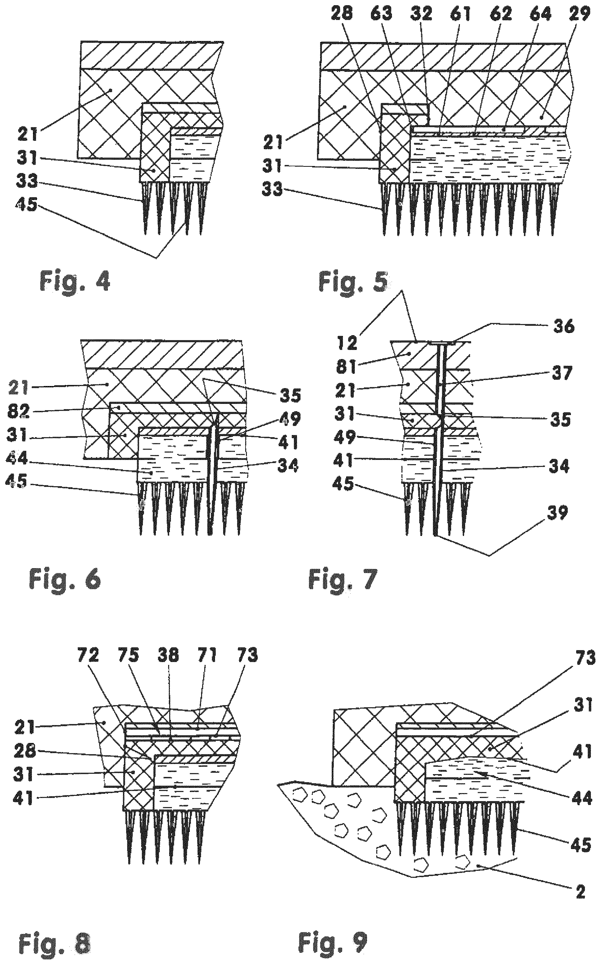

Es ist auch denkbar, Nadeln (33) am Vorschubelement (31) anzuordnen, vgl. Figur 4. Diese haben beispielsweise die gleichen Innen- und Außenabmessungen wie Mikronadeln (45). Die Nadeln (33) können auch am Nadelträger (43) außerhalb des Verteilkanals (48) befestigt sein.

Beim Einsatz des transdermalen therapeutischen Systems (10) wird dieses mit den Nadeln (45) auf die Haut (2) des Patienten aufgesetzt und so weit angedrückt, bis die Auflagefläche (24) auf der Haut (2) des Patienten anliegt, vgl. Figur 2. Die adhäsive Schicht (22) verklebt hierbei die Haut (2) mit dem transdermalen therapeutischen System (10). Das Vorschubelement (31) kann dabei die Haut (2) des Patienten kontaktieren. Die Nadeln (45) sind in die Haut (2) des Patienten eingedrungen. Aufgrund ihrer geringen Länge durchdringen sie im Wesentlichen die äußere Hautschicht (Stratum Corneum) und enden außerhalb der Nervenendungen der tieferen Hautschichten, so dass der Patient im Wesentlichen keinen Schmerz bei de Applikation verspürt.

Das Vorschubelement (31) nimmt Feuchtigkeit von der Haut (2) auf. Das Volumen des Quellkörpers (31) vergrößert sich, vgl. Figur 3. Gegebenenfalls kann, wie in der Figur 4 dargestellt, Flüssigkeit aus dem Bereich unterhalb der Hornhaut aufgenommen werden. Da der Systemträger (21) aufgrund seiner höheren Steifigkeit die freie Verformung des Quellkörpers (31) verhindert oder zumindest stark reduziert, kann der Quellkörper (31) im Wesentlichen nur in die Richtung des Wirkstoffreservoirs (41) expandieren. Das elastisch verformbare Wirkstoffreservoir (41) wird verformt und komprimiert. Hierbei wird Wirkstoff (44) aus dem Innenraum (46) des Wirkstoffreservoirs (41) in den Nadelträger (43) und in die Nadeln (45) verdrängt. Aus den Nadeln (45) wird der Wirkstoff (44) in die Haut (2) des Patienten gefördert. Bei weiterer Flüssigkeitsaufnahme wird das Wirkstoffreservoir (41) weiter verformt. Hierdurch wird zunehmend Wirkstoff (44) in die Haut (2) des Patienten gepresst. Während der Flüssigkeitsaufnahme wird die Flüssigkeit im Quellkörper (31) gelagert und nicht abgegeben.

Die Wirkstoffabgabe ist beendet, wenn entweder der Quellkörper (31) auf sein maximales Volumen expandiert ist oder wenn sämtlicher Wirkstoff (44) aus dem Wirkstoffreservoir (41) in Richtung der Haut (2) verdrängt ist.

In der Ausführungsform der Figur 5 ragt der Systemträger (21) mit einem Stempel (29) in den Innenraum (28). Dieser Stempel hat beispielsweise einen quaderförmigen Querschnitt und begrenzt das seitlich an ihm anliegende Vorschubelement (31). Das z.B. mattenförmige Vorschubelement (31) hat in diesem Ausführungsbeispiel eine zentrale Ausnehmung (32), die den Stempel (29) umgreift. In der Darstellung der Figur 5 ist oberhalb des Wirkstoffreservoirs (41) die Druckplatte (61) angeordnet. Die Druckplatte (61) umfasst eine Platte (62) mit einem Verstärkungsrand (63). Im Ausführungsbeispiel sind an der Oberseite der Platte (62) außerdem Diagonalrippen (64) angeordnet, die sich in der Mitte der Oberseite der Druckplatte (61) schneiden. Hiermit ist die Druckplatte (61) verwindungssteif und biegesteif ausgebildet. Gegebenenfalls kann die Druckplatte (61) zusätzlich in einem z.B. vertikal angeordneten Führungsbolzen geführt sein.

Beim Einsatz des transdermalen therapeutischen Systems (10) wird auch in diesem Ausführungsbeispiel Flüssigkeit von der Hautoberfläche oder aus der Haut (2) mittels des Vorschubelements (31) aufgenommen. Der mittels der Flüssigkeitsaufnahme expandierende Quellkörper (31) drückt die Druckplatte (61) in der Darstellung der Figur 5 nach unten. Der Quellkörper (31) wird hierbei am Systemträger (21) und am Stempel (29) geführt. Die Druckplatte (61) wird zumindest annähernd parallel verschoben und presst das Wirkstoffreservoir (41) zusammen. Bei einer z.B. mittels eines Führungszapfens geführten Druckplatte (61) verhindert die Führung zusätzlich ein Verkanten. Der Wirkstoff (44) wird weitgehend gleichmäßig aus dem Innenraum (46) in die Nadeln (45) und in die Haut (2) verdrängt.

Die Figur 6 zeigt ein transdermales therapeutisches System (10) mit zusätzlichen Hohlnadeln (34). Diese Hohlnadeln (34) sind parallel zu den Nadeln (45) angeordnet, die den Wirkstoff (44) in die Haut (2) fördern. Beispielsweise beträgt die Anzahl der Hohlnadeln (34) ein Fünftel der Anzahl der wirkstofffördernden Nadeln (45). Die Hohlnadeln (34), deren Spitzen (39) im Ausführungsbeispiel die gleichen Abmessungen wie die wirkstoffördernden Nadeln (45) haben, durchdringen das Wirkstoffreservoir (41) z.B. in abgedichteten Durchbrüchen (49). Die Hohlnadeln (34) durchdringen das Vorschubelement (31) und stützen sich in der Darstellung der Figur 6 am Schutzlayer (82) im Systemträger (21) ab. Im Bereich des Vorschubelements (31) haben die Hohlnadeln (34) ihre Umfangsfläche durchdringende Durchbrüche (35). Hiermit ist der zentrale Bereich des Vorschubelements (31) mittels der Hohlnadeln (34) mit der Umgebung (1) verbunden. Der Abstand des Durchbruchs (35) von der Nadelspitze (39) ist kleiner als der Kehrwert des Durchmessers des Hohlraums (37) in Millimetern, multipliziert mit einer Fläche von 25 Quadratmillimetern. Beispielsweise bei einem Durchmesser des Hohlraums (37) von 0,5 Millimetern ergibt sich der maximale Abstand zu 50 Millimetern.

Beim Betrieb des transdermalen therapeutischen Systems (10) steigt die Flüssigkeit aus der Haut (2) beispielsweise aufgrund der Kapillarwirkung durch die Hohlnadeln (34) auf und gelangt in das Vorschubelement (31). Die hier eindringende Flüssigkeit bewirkt ein zusätzliches Aufquellen des Quellkörpers (31) in den an die Hohlnadeln (34) angrenzenden Bereichen. Beispielsweise kann der zentrale Abschnitt des Wirkstoffreservoirs (41) frühzeitiger komprimiert werden als bei der Ausführungsform nach den Figuren 1 - 3.

In der Darstellung der Figur 7 durchdringt die Hohlnadel (34) den Systemträger (21). Beispielsweise an der Außenseite des Abdeckungslayers (81) ist eine semipermeable Membran (36) angeordnet, die den Hohlraum (37) der Hohlnadel (34) auf der Handhabungsseite (12) verschließt. Diese semipermeble Membran (36) erlaubt den Durchtritt von Gas und/oder Flüssigkeit aus dem Hohlraum (37) in die Umgebung (1). Das Eintreten dieser Stoffe aus der Umgebung (1) in den Hohlraum (37) ist jedoch blockiert. Anstatt der semipermeablen Membran (36) kann auch ein Rückschlagventil, etc. vorgesehen sein.

Die Funktion des in der Figur 7 dargestellten transdermalen therapeutischen Systems (10) ist ähnlich der Funktion des in der Figur 6 dargestellten Systems (10). Die beim Aufsteigen der Flüssigkeit aus der Kapillare (37) verdrängte Luft kann hier durch die semipermeable Membran (36) hindurch in die Umgebung (1) entweichen.

Die Figur 8 und 9 zeigen ein transdermales therapeutisches System (10) mit einem integrierten Flüssigkeitsreservoir (71), z.B. einem Wasserreservoir (71). Das Wasserreservoir (71) ist im Innenraum (28) des Systemträgers (21) zwischen dem Vorschubelement (31) und dem Systemträger (21) angeordnet. Es umfasst einen z.B. aufreißbaren Mantel (72). Zwischen desm Wasserreservoir (71) und dem Vorschubelement (31) ist in den Darstellungen der Figuren 8 und 9 ein Gitter (73) angeordnet. Dieses Gitter (73) ist beispielsweise im Systemträger (21) verankert.

Das Vorschubelement (31) trägt auf der dem Wasserreservoir (71) zugewandten Seite Aufreißdorne (38), die zunächst beispielsweise um wenige zehntel Millimeter beabstandet sind vom Wasserreservoir (71).

Beim Aufsetzen des transdermalen therapeutischen Systems (10) auf die Haut (2) werden die Nadeln (45) und das Wirkstoffreservoir (41) gegen den Systemträger (21) verschoben. Das Wirkstoffreservoir (41) verschiebt das im Systemträger (21) geführte Vorschubelement (31) in Richtung des Wasserreservoirs (71). Hierbei kontaktieren die Aufreißdorne (38) den Mantel (72) des Wasserreservoirs (71) und reißen den Mantel (72) des Wasserreservoirs (71) auf, vgl. Figur 9. Das aus dem Wasserreservoir (71) austretende Wasser (75) dringt in das Vorschubelement (31) ein und bewirkt sein Aufquellen. Gegebenenfalls kann das Wasser (75) in das Vorschubelement (31) einspritzen. Das Gitter (73) verhindert hierbei eine Expansion des Quellkörpers (31) in der Richtung des Systemträgers (21). Der Quellkörper (31) komprimiert das Wirkstoffreservoir (41). Der Wirkstoff (44) wird aus dem Wirkstoffreservoir (41) durch die Nadeln (45) hindurch in die Haut (2) gepresst.

Es ist auch denkbar, rohrförmige Kapillare im Vorschubelement (31) anzuordnen. Hiermit kann, zusätzlich zu der Förderwirkung des Werkstoffs des Vorschubelements (31), Flüssigkeit in den zentralen Bereich des Vorschubelements (31) gefördert werden. Auch eine Flüssigkeitsführung mittels der Zwischenschicht (61) ist denkbar. Gegebenenfalls kann damit das Aufquellen des Quellkörpers (31) gesteuert werden.

Selbstverständlich ist es auch denkbar, die verschiedenen genannten Ausführungsformen miteinander zu kombinieren.

### Bezugszeichenliste:

- 1: Umgebung
- 2: Haut

- 10: Transdermales therapeutisches System
- 11: Anwendungsseite
- 12: Handhabungsseite

- 21: Systemträger
- 22: adhäsive Schicht
- 23: Tragteil
- 24: Auflagefläche
- 25: Schale
- 26: Deckenteil
- 27: Wandteile
- 28: Innenraum
- 29: Stempel

- 31: Vorschubelement, Quellkörper
- 32: Ausnehmung
- 33: Nadeln, hohle Nadel
- 34: Hohlnadeln
- 35: Durchbrüche
- 36: semipermeable Membran, Rückschlagventil
- 37: Hohlraum, Kapillare
- 38: Aufreißdorne
- 39: Nadelspitze

- 41: Wirkstoffreservoir
- 42: Wandungen
- 43: Nadelträger
- 44: Wirkstoff, Wirkstoffzubereitung
- 45: Nadeln, Mikronadeln
- 46: Innenraum
- 47: Durchgangsbohrung
- 48: Verteilkanal
- 49: Durchbrüche

- 61: Druckplatte, Zwischenschicht, wasseraufnehmende Schicht
- 62: Platte
- 63: Verstärkungsrand
- 64: Diagonalrippen

- 71: Flüssigkeitsreservoir. Wasserreservoir
- 72: Mantel
- 73: Gitter
- 75: Wasser

- 81: Abdeckungslayer
- 82: Schutzlayer

## Patentansprüche

1. Transdermales therapeutisches System (10) mit einem Systemträger (21), mit mindestens einem in den Systemträger (21) eingebetteten Vorschubelement (31) und mit mindestens einem zumindest bereichsweise elastisch verformbaren, in das Vorschubelement (31) eingebetteten Wirkstoffreservoir (41), wobei der Systemträger (21) auf der der Haut des Patienten zuwendbaren Anwendungsseite (11) des transdermalen therapeutischen Systems (10) eine Auflagefläche (24) aufweist, **dadurch gekennzeichnet dass** der Systemträger (21) als konkaver Körper aufgebaut ist, der fünfseitig einen Innenraum (28) des Systemträgers (21) begrenzt, wobei im Innenraum (28) das mit der Haut (2) des Patienten kontaktierbare Vorschubelement (31) angeordnet ist, wobei das Wirkstoffreservoir (41) über die Auflagefläche (24) aus dem Vorschubelement (31) hervorsteht und wobei das Vorschubelement (31) einen mittels Flüssigkeitsaufnahme expandierbaren Quellkörper (31) umfasst, wobei der Systemträger (21) zumindest bereichsweise verformungssteif ausgebildet ist.

2. Transdermales therapeutisches System (10) nach Anspruch 1,
**dadurch gekennzeichnet,**
**dass** das Wirkstoffreservoir (41) hohle Mikronadeln (45) aufweist, die den Innenraum (46) des Wirkstoffreservoirs (41) auf der Anwendungsseite (11) mit der Umgebung (1) verbinden.

3. Transdermales therapeutisches System (10) nach Anspruch 2,
**dadurch gekennzeichnet,**
**dass** die Mikronadeln (45) porös und/oder quellbar ausgebildet sind.

4. Transdermales therapeutisches System (10) nach Anspruch 1,
**dadurch gekennzeichnet,**
**dass** der Systemträger (21) zumindest auf der Anwendungsseite (11) eine adhäsive Schicht (22) umfasst.

5. Transdermales therapeutisches System (10) nach Anspruch 1,
**dadurch gekennzeichnet,**
**dass** das Wirkstoffreservoir (41) eine flüssige oder verflüssigbare Wirkstoffzubereitung (44) umfasst.

6. Transdermales therapeutisches System (10) nach Anspruch 1,
**dadurch gekennzeichnet,**
**dass** zwischen dem Quellkörper (31) und dem Wirkstoffreservoir (41) eine Zwischenschicht (61) angeordnet ist.

7. Transdermales therapeutisches System (10) nach Anspruch 1,
**dadurch gekennzeichnet,**
**dass** mindestens eine hohle Nadel (33, 34) den Quellkörper (31) mit der Umgebung (1) auf der Anwendungsseite (11) verbindet.

8. Transdermales therapeutisches System (10) nach Anspruch 7,
**dadurch gekennzeichnet,**
**dass** auf der der Anwendungsseite (11) abgewandten Handhabungsseite (12) eine semipermeable Membran (36) die hohle Nadel (34) verschließt.

## Claims

1. A transdermal therapeutic system (10) having a system support (21), having at least one advancing element (31) which is embedded in the system support (21) and having at least one active substance reservoir (41) which is elastically deformable in at least some regions and which is embedded in the advancing element (31), wherein the system support (21) comprises a bearing surface (24) on an application side (11) of the transdermal therapeutic system (10) which can be faced towards the skin, **characterized in that** the system support (21) is configured as a concave body which delimits an interior (28) of the system support (21) on five sides, wherein the advancing element (31) which can come into contact with the skin (2) of the patient is disposed in the interior (28), wherein the active substance reservoir (41) protrudes out of the advancing element (31) from the bearing surface (24) and wherein the advancing element (31) comprises a swelling body (31) which can be expanded by liquid uptake, wherein the system support (21) is configured so as to be resistant to deformation at least in some regions.

2. The transdermal therapeutic system (10) as claimed in claim 1, **characterized in that** the active substance reservoir (41) comprises hollow micro-needles (45) which connect the interior (46) of the active substance reservoir (41) to the environment (1) on the application side (11).

3. The transdermal therapeutic system (10) as claimed in claim 2, **characterized in that** the micro-needles (45) are porous and/or swellable in configuration.

4. The transdermal therapeutic system (10) as claimed in claim 1, **characterized in that** the system support (21) comprises an adhesive layer (22) on at least the application side (11).

5. The transdermal therapeutic system (10) as claimed in claim 1, **characterized in that** the active substance reservoir (41) comprises a liquid or liquefiable active substance formulation (44).

6. The transdermal therapeutic system (10) as claimed in claim 1, **characterized in that** an intermediate layer (61) is disposed between the swelling body (31) and the active substance reservoir (41).

7. The transdermal therapeutic system (10) as claimed in claim 1, **characterized in that** at least one hollow needle (33, 34) connects the swelling body (31) to the environment (1) on the application side (11).

8. The transdermal therapeutic system (10) as claimed in claim 7, **characterized in that** a semipermeable membrane (36) closes the hollow needle (34) on a handling side (12) facing away from the application side (11).

## Revendications

1. Système thérapeutique transdermique (10) comportant un support de système (21) avec au moins un élément d'avancement (31) incorporé dans le support de système (21) et au moins un réservoir de substance active (41) élastiquement déformable au moins par endroits, incorporé dans l'élément d'avancement (31), le support de système (21) présentant une surface d'appui (24) sur le côté de l'application (11), orientable vers la peau du patient, du système thérapeutique transdermique (10),
**caractérisé en ce**
**que** le support de système (21) est réalisé sous forme d'un corps concave qui délimite un espace intérieur (28) du support de système (21) sur cinq faces, l'élément d'avancement (31) qui peut être mis en contact avec la peau (2) du patient étant disposé dans l'espace intérieur (28), le réservoir de substance active (41) dépassant l'élément d'avancement (31) en passant par-dessus la surface d'appui (24) et l'élément d'avancement (31) comprenant un corps dilatable (31) qui est extensible par absorption de liquides, le support de système (21) étant résistant à la déformation au moins par endroits.

2. Système thérapeutique transdermique (10) selon la revendication 1, **caractérisé en ce que** le réservoir de la substance active (41) comporte des micro-aiguilles creuses (45) qui relient l'espace intérieur du réservoir de la substance active (41) sur le côté de l'application (11) à l'espace environnant (1).

3. Système thérapeutique transdermique (10) selon la revendication 2, **caractérisé en ce que** les micro-aiguilles (45) sont réalisées sous forme poreuse et/ou gonflable.

4. Système thérapeutique transdermique (10) selon la revendication 1, **caractérisé en ce que** le support de système (21) comporte au moins une couche adhésive (22) située sur le côté de l'application (11).

5. Système thérapeutique transdermique (10) selon la revendication 1, **caractérisé en ce que** le réservoir (41) de la substance active comporte une préparation active (44) liquide ou liquéfiable.

6. Système thérapeutique transdermique (10) selon la revendication 1, **caractérisé en ce qu'**une couche intermédiaire (61) est placée entre le corps dilatable (31) et le réservoir de la substance active (41).

7. Système thérapeutique transdermique (10) selon la revendication 1, caractérisé qu'au moins une aiguille creuse (33, 34) relie le corps dilatable (31) à l'espace environnant (1) sur le côté de l'application (11).

8. Système thérapeutique transdermique (10) selon la revendication 7, **caractérisé en ce qu'**une membrane semi-perméable (36) ferme l'aiguille creuse (34) sur le côté de la manipulation (12), opposée au côté de l'application (11).
